# EUROPEAN PATENT SPECIFICATION

(11) **EP 3 558 215 B1**
(45) Date of publication and mention of the grant of the patent: **17.07.2024**
(21) Application number: 16924222.9
(22) Date of filing: 23.12.2016
(51) Int. Cl.: A61K 8/46, A61Q 19/00, A61K 8/34, A61K 8/33, A61K 8/42

(54) **COMPOSITION HAVING ALPHA-CRYSTALLINE PHASE**
ZUSAMMENSETZUNG MIT ALPHA-KRISTALLINER PHASE
COMPOSITION PRÉSENTANT UNE PHASE ALPHA-CRISTALLINE

(43) Date of publication of application: 30.10.2019
(73) Proprietor: L'OREAL, 75008 Paris (FR); Song, Ji, Shanghai 201206 (CN); Weng, Xiaomin, Shanghai 201206 (CN); Lu, Haiquan, Shanghai 201206 (CN)
(72) Inventor: SONG, Ji, Shanghai 201206 (CN); WENG, Xiaomin, Shanghai 201206 (CN); LU, Haiquan, Shanghai 201206 (CN)
(74) Representative: Casalonga
(86) International application number: PCT/CN2016/111643
(87) International publication number: WO 2018/112867

(56) References cited:
- EP-A1- 2 335 681
- EP-A1- 2 548 549
- WO-A1-2015/190306
- WO-A1-2017/070940
- CN-A- 102 028 647
- US-A1- 2012 164 094
- US-A1- 2013 005 835

## Description

### TECHNICAL FIELD

The present invention relates to a composition having an α-crystalline phase. It relates more particularly to a composition having an α-crystalline phase formed by oily globules provided with a lamellar crystal coating and dispersed in an aqueous phase. The present invention also relates to a process for caring for and/or making up keratin materials and in particular the skin.

### BACKGROUND

In the cosmetic art it is known to formulate compositions with lamellar structures, such as α-gel or liquid crystal structures, for improving the stability of cosmetic compositions. Moreover, thanks to the lamellar structure, i.e., repeating bilayer structures, the cosmetic compositions are capable of providing to the skin a good retention of water, and therefore a long-lasting moistness. Various types of surfactants were used for stabilizing the α-gel structure and providing to the skin a water retention effect.

For example, US 2014/0100276 discloses an emulsion composition which allows water to remain in the skin for a long period of time, wherein the emulsion presents a lamellar structure, which is, in particular in form of α-gel.

Other emulsion compositions involving an α-gel are known from EP 2 548 549 and WO 2015/190306.

Mentions can also be made to the known products on the market, using combinations of surfactants to formulate creams with liquid crystal structure.

On the other hand, the optical effect of a cosmetic product is an important aspect. Consumers are now more and more in favour of cosmetic compositions, especially compositions caring for the skin, with optical effect in order to make the skin beautiful, or to cover the imperfections on the skin. In particular, consumers are always in search of a cosmetic product with a haze (blurring) effect.

However, among all the known arts, in order to achieve some haze (blurring) effect, high haze (blurring) effect raw materials, such as whitening charges or silicone elastomers are used, which will result in dry squeaky or artificial slippery sensation.

Also it is difficult to solve these technical problems by simply formulating whitening charges or silicone elastomers into a lamellar structure composition, with the aim of preparing a composition for treating keratin materials and remain stable, in particular under high temperatures, such as 45 °C.

Therefore there is a need to formulate a composition with a lamellar structure, and delivering a blurring effect to keratin materials, such as the skin.

Besides, there is a need to formulate such a composition as mentioned above, with a good skin feeling, such as water retention, moisturizing feeling.

There is a need to formulate a composition for caring for / making up keratin materials with haze (blurring) effect while avoid dry squeaky or artificial slippery sensation, wherein the composition is stable, in particular under high temperatures, such as 45 °C.

### SUMMARY OF THE INVENTION

The applicant found that such a need can be met by formulating a composition having an α-crystalline phase, with a combination of oil(s), specific solid fatty alcohol(s), and specific anionic surfactant(s).

More specifically, the present invention relates to a composition having an α-crystalline phase, comprising at least one continuous aqueous phase and at least one dispersed oily phase, and comprising:
(a) at least one oil;
(b) greater than or equal to 3% by weight of at least one solid fatty alcohol relative to the total weight of the composition; and
(c) at least one anionic surfactant of fatty acid amide sulfonate salts,

wherein the weight ratio of the solid fatty alcohol (b)/the anionic surfactant (c) ranges from 15 to 40,
wherein the α-crystalline phase having a surface area moment mean diameter D[3,2] ranging from 1µm to 35µm.

The present invention also relates to a process for caring for/making up keratin materials, in particular the skin, comprising the application to the keratin materials of the composition according to the present invention.

The present invention also relates to the use of the composition according to the present invention for caring for keratin materials, in particular the skin.

The diameter D[3,2] of the α-crystalline phase according to the present invention is a parameter for referring to the surface weighted mean, also known as the surface area moment mean diameter or Sauter mean. The diameter of the α-crystalline phase may be measured by static light scattering using a commercial granulometer such as the MasterSizer 3000 machine from Malvern.

The composition of the present invention has a blurring effect upon application.

The blurring effect is characterized by instrumental measurement of haze of the composition according to the present invention.

In particular, the haze value is measured by the machine Haze-gard Plus sold by the company BYK.

The measurement is conducted by preparing a film the composition of the present invention with a thickness of 25µm using the machine Elcometer 4340 Automatic Film Applicator on a transparent film (BYK Byko-charts clear polyester-2mil #2871).

By "keratin materials" we intend to mean human keratin materials and more specifically the skin.

Other subjects and characteristics, aspects and advantages of the present invention will emerge even more clearly on reading the description and the examples that follows. In that which follows and unless otherwise indicated, the limits of a range of values are included within this range, in particular in the expressions "of between" and "ranging from ... to ...".

Moreover, the expression "at least one" used in the present description is equivalent to the expression "one or more".

### DETAILED DESCRIPTION OF THE PREFERRED EMBODIMENTS

### (a) oil

According to the present invention, the composition comprises at least one oil.

The term "oil" refers to any fatty body in liquid form at room temperature (20-25°C) and atmospheric pressure. These oils may be of animal, plant, mineral or synthetic origin. The oils may be volatile or non-volatile.

The term "volatile oil" refers to any non-aqueous medium capable of evaporating from the skin or lips, in less than one hour, at room temperature (20-25°C) and atmospheric pressure (760 mmHg).

More specifically, the volatile oil is a volatile cosmetic oil, liquid at room temperature. More specifically, a volatile oil has an evaporation rate of between 0.01 and 200mg/cm²/min, inclusive.

The term "non-volatile oil" is intended to mean an oil remaining on the skin or keratin fiber at ambient temperature and atmospheric pressure.

More specifically, a non-volatile oil has an evaporation rate strictly below 0.01 mg/cm²/min.

To measure this evaporation rate, 15g of oil or a mixture of oils to be tested are introduced into a crystallizer, 7cm in diameter, placed on a scale located in a large 0.3m³ chamber temperature-controlled at a temperature of 25°C, and humidity-controlled with a relative humidity of 50%. The liquid is left to evaporate freely, without stirring, by providing ventilation with a fan (PAPST-MOTOREN, reference 8550 N, rotating at 2700 rpm) positioned vertically above the crystallizer containing the solvent, with the blades directed toward the crystallizer and at a distance of 20cm from the base of the crystallizer. The mass of oil remaining in the crystallizer is measured at regular intervals. The evaporation rates are expressed in mg of oil evaporated per surface area unit (cm²) and per time unit (minute).

The oils that are suitable for the present invention may be hydrocarbon-based, silicone-based or fluorine-based.

According to the present invention, the term "silicone oil" refers to an oil including at least one silicon atom, and in particular at least on Si-O group.

The term "fluorine oil" refers to an oil including at least one fluorine atom.

The term "hydrocarbon-based oil"refers to an oil containing primarily hydrogen and carbon atoms.

The oils may optionally include oxygen, nitrogen, sulfur and/or phosphorus atoms, for example, in the form of hydroxyl or acid radicals.

More preferably, the composition of the present invention comprises hydrocarbon oil(s). Specifically, the volatile oils may be chosen from hydrocarbon oils having 8 to 16 carbon atoms, and in particular branched C₈-C₁₆ alkanes (also called isoparaffins or isoalkanes), such as isododecane (also called 2,2,4,4,6-pentamethylheptane), isodecane, isohexadecane, and, for example, the oils sold under the trade names ISOPARS^{®} or PERMETHYLS^{®}.

It is also possible to cite, as a hydrocarbon volatile oil, linear C₉-C₁₇ alkanes, such as dodecane (C₁₂) and tetradecane (C₁₄), sold respectively under the names PARAFOL^{®} 12-97 and PARAFOL^{®} 14-97 (Sasol), and, as alkanes obtained according to the method described in the international application WO 2007/068371 A1, such as the undecane (C₁₁) and tridecane (C₁₃) mixture sold under the name CETIOL^{®} UT (Cognis).

The non-volatile oils may, in particular, be chosen from among the non-volatile hydrocarbon oils.

It is possible to cite, as a non-volatile hydrocarbon oil:
- hydrocarbon oils of animal origin, such as perhydrosqualene,
- hydrocarbon oils of plant origin, such as phytostearyl esters, for instance phytostearyl oleate, phytostearyl isostearate and lauroyl/octyldodecyl/phytostearyl glutamate (AJINOMOTO, ELDEW PS203), diesters such as diisopropyl sebacate, triglycerides constituted of fatty acid esters of glycerol, in particular in which the fatty acids may have chain lengths ranging from C₄ to C₃₆, and in particular from C₁₈ to C₃₆, it being possible for these oils to be linear or branched, and saturated or unsaturated; these oils may in particular be heptanoic or octanoic triglycerides, shea oil, alfalfa oil, poppy seed oil, pumpkin oil, millet oil, barley oil, quinoa oil, rye oil, candlenut oil, passionflower oil, shea butter, aloe oil, sweet almond oil, peach kernel oil, groundnut oil, argan oil, avocado oil, baobab oil, barrage oil, broccoli oil, calendula oil, camelina oil, canola oil, carrot oil, safflower oil, hemp oil, rapeseed oil, cotton seed oil, coconut oil, marrow seed oil, wheat germ oil, jojoba oil, lily oil, macadamia oil, corn oil, meadowfoam oil, St. John's Wort oil, monoi oil, hazelnut oil, apricot kernel oil, nut oil, olive oil, evening primrose oil, palm oil, blackcurrant seed oil, kiwi seed oil, grape seed oil, pistachio oil, pumpkin oil, winter squash oil, quinoa oil, musk rose oil, sesame oil, soya oil, sunflower oil, castor oil and watermelon oil, and mixtures thereof, or alternatively caprylic/capric acid triglycerides, for instance those sold by the STEARINERIES DUBOIS company or those sold under the names MIGLYOL 810^{®}, 812^{®} and 818^{®} by the DYNAMIT NOBEL company,
- linear or branched hydrocarbons of mineral or synthetic origin, such as liquid paraffins and derivatives thereof, petroleum jelly, polydecenes, polybutenes, hydrogenated polyisobutene such as Parleam, squalane;
- synthetic ethers having from 10 to 40 carbon atoms;
- synthetic esters, for instance oils of formula R₁COOR₂, in which R₁ represents a linear or branched fatty acid residue containing from 1 to 40 carbon atoms, and R₂ represents a hydrocarbon-based chain, in particular a branched chain, containing from 1 to 40 carbon atoms provided that R₁ or R₂ is greater than or equal to 10. The esters may in particular be selected from esters of fatty acid and of alcohol, for instance: cetostearyl octanoate, isopropyl alcohol esters, such as isopropyl myristate, isopropyl palmitate, ethyl palmitate, 2-ethylhexyl palmitate, isopropyl stearate or isostearate, isostearyl isostearate, octyl stearate, hydroxylated esters, for instance isostearyl lactacte, octyl hydroxystearate, diisopropyl adipate, heptanoates, and especially isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate and palmitate, alkyl benzoate, polyethylene glycol diheptanoate, propylene glycol 2-diethylhexanoate, and mixtures thereof, C₁₂-C₁₅ alkyl benzoates, hexyl laurate, neopentanoic acid esters, for instance isodecyl neopentanoate, isotridecyl neopentanoate, isostearyl neopentanoate, or octyldodecyl neopentanoate, isononanoic acid esters, for instance isononyl isononanoate, isotridecyl isononanoate and octyl isononanoate, hydroxylated esters such as isostearyl lactate and diisostearyl malate;
- polyol esters and pentaerythritol esters, for instance dipentaerythrityl tetrahydroxystearate/tetraisostearate,
- esters of diol dimers and diacid dimers, such as Lusplan DD-DA5^{®} and Lusplan DD-DA7^{®}, sold by the NIPPON FINE CHEMICAL company and described in the application US 2004-175338,
- copolymers of a diol dimer and of a diacid dimer and esters thereof, such as copolymers of dilinoleyl diol dimers/dilinoleic dimers and esters thereof, for instance Plandool-G,
- copolymers of polyols and of diacid dimers, and esters thereof, such as Hailuscent ISDA, or the copolymer of dilinoleic acid/butanediol,
- fatty alcohols that are liquid at ambient temperature, with a branched and/or unsaturated carbon chain having from 12 to 26 carbon atoms, for instance 2-octyldodecanol, isostearyl alcohol, oleyl alcohol, 2-hexyldecanol, 2-butyloctanol and 2-undecylpentadecanol,
- C₁₂-C₂₂, higher fatty acids, such as oleic acid, linoleic acid or linolenic acid, and mixtures thereof, and,
- dialkyl carbonates of the following formula (I): 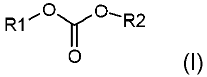 wherein R₁ and R₂, identical or different, each represents a linear or branched hydrocarbon chain comprising from 3 to 30 carbon atoms.

Preferably, according to an embodiment, in formula (I), R₁ and R₂, which are identical, represent a linear hydrocarbon chain comprising from 3 to 8 carbon atoms.

More preferably, the dialkyl carbonate is dicaprylyl carbonate.

In one embodiment, the carbonate is caprylyl carbonate. the two alkyl chains possibly being identical such as the dicaprylyl carbonate sold under the name CETIOL CC^{®}, by COGNIS.
- oils of higher molar mass having in particular a molar mass ranging from approximately 400 to approximately 10,000 g/mol, in particular from approximately 650 to approximately 10,000 g/mol, in particular from approximately 750 to approximately 7500 g/mol, and more particularly ranging from approximately 1000 to approximately 5000 g/mol. As oils of higher molar mass that can be used in the present invention, mention may in particular be made of the oils selected from:
   - lipophilic polymers,
   - linear fatty acid esters having a total carbon number ranging from 35 to 70,
   - hydroxylated esters,
   - aromatic esters,
   - esters of C₂₄-C₂₈ branched fatty acids or fatty alcohols,
   - oils of plant origin,
   - and mixtures thereof; and
- mixtures thereof.

In one embodiment, the composition of the present invention comprises, as oil, dicaprylyl carbonate.

According to a preferred embodiment, the at least one oil (a) is present in an amount ranging from 0.1% to 15% by weight, preferably ranging from 1% to 10% by weight, relative to the total weight of the composition.

### (b) Solid fatty alcohol

The composition of the present invention comprises greater than or equal to 3% by weight of at least one solid fatty alcohol, relative to the total weight of the composition. The term "fatty alcohol" means a long-chain aliphatic alcohol comprising from 8 to 40 carbon atoms, preferably from 12 to 34 or even from 12 to 30 carbon atoms, and comprising at least one hydroxyl group OH. These fatty alcohols are neither oxyalkylenated nor glycerolated.

The "solid fatty alcohols" are solid at room temperature (25°C) and at atmospheric pressure (780 mmHg or 1 atm.); they are water-insoluble, i.e. they have a solubility in water of less than 1% by mass and preferably less than 0.5% by weight.

Preferably, the solid fatty alcohols are of structure R-OH with R denoting a saturated or unsaturated, linear alkyl group, optionally substituted with one or more hydroxyl groups, comprising from 14 to 30 carbon atoms.

Preferably, the solid fatty alcohols used in the present invention are selected from fatty alcohols having from 14 to 30 carbon atoms.

Mentions may be made of, as solid fatty alcohols, myristyl alcohol (1-tetradecanol), cetyl alcohol (1-hexadecanol), palmitoleyl alcohol (cis-9-hexadecen-1-ol), stearyl alcohol (1-octadecanol), arachidyl alcohol (1-eicosanol), behenyl alcohol (1-docosanol), erucyl alcohol (cis-13-docosen-1-ol), lignoceryl alcohol (1-tetracosanol), ceryl alcohol (1-hexacosanol), myricyl alcohol, or melissyl alcohol (1-triacontanol).

More preferably, according to the present invention, the solid fatty alcohol is selected from alcohols having from 14 to 22 carbon atoms, such as cetyl alcohol (1-hexadecanol), stearyl alcohol (1-octadecanol), arachidyl alcohol (1-eicosanol), behenyl alcohol (1-docosanol), or a mixture thereof.

More particularly, the solid fatty alcohol are selected from cetyl alcohol (1-hexadecanol), stearyl alcohol (1-octadecanol), arachidyl alcohol (1-eicosanol), behenyl alcohol (1-docosanol), or a mixture thereof.

The fatty alcohols may be mixtures, which mean, for example, that several species may coexist in a commercial product, especially of different chain lengths, in the form of a mixture.

Mentions of such mixture of fatty alcohols may be made of cetearyl alcohol sold under the name Lanette O Or by BASF, wherein containing predominantly a mixture of cetyl alcohol and stearyl alcohol.

According to one embodiment, the solid fatty alcohol (b) is present in an amount greater than or equal to 3% by weight, preferably from 3% to 15% by weight, more preferably from 3% to 10% by weight, relative to the total weight of the composition.

### (c) Anionic surfactant

The composition of the present invention comprises at least one anionic surfactant of fatty acid amide sulfonate salt.

In one embodiment, the anionic surfactant is at least one amino acid surfactant of the following formula (II): 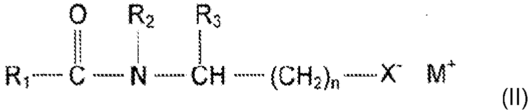 wherein:
R₁ is a saturated or unsaturated, straight or branched alkyl chain with 7 to 17 carbon atoms atoms,
R₂ is H or a methyl,
R₃ is H, COO⁻ M⁺, CH₂COO- M or COOH,
n is 0 to 2,
X is COO⁻ or SO₃ ⁻ and
M is independent from each other H, sodium or potassium and a sorbitan surfactant.
Such anionic surfactants are those described in EP2335681.

Suitably amino acid surfactant types are taurate, glutamate, alanin or alaninate, sarcosinate, aspartate surfactants, and mixtures thereof.

Preferred are taurate, glutamate and sarcosinate surfactants and mixtures thereof. More preferred are taurates and glutamates and most preferred is glutamate type surfactants.

Suitable taurate surfactants are according to the general formula (III) 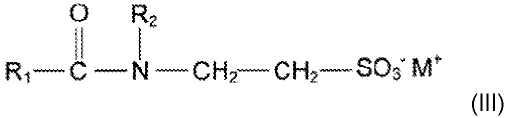 wherein
R₁ is preferably a saturated or unsaturated, straight or branched alkyl chain with 7 to 17 carbon atoms, and more preferably 9 to 13 carbon atoms,
R₂ is H or methyl,
and M is H, sodium or potassium.

Suitable examples are potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, and sodium methyl stearoyl taurate and mixtures thereof. Preferred are potassium cocoyl taurate, potassium methyl cocoyl taurate, sodium caproyl methyl taurate, sodium cocoyl taurate, sodium lauroyl taurate, sodium methyl cocoyl taurate and sodium methyl lauroyl taurate and mixtures thereof. More preferred are potassium cocoyl taurate, potassium methyl cocoyl taurate, sodium cocoyl taurate, sodium lauroyl taurate, sodium methyl cocoyl taurate and sodium methyl lauroyl taurate and mixtures thereof.

It should be noted that compositions of the present invention can also comprise mixture of several type of amino acid surfactants such as mixture of glutamate and taurate surfactants, or mixture of taurate, glutamate and sarcosinate surfactants etc.

With the term amino acid surfactant especially those surfactants are meant derived from taurate, glucamate, alanin or alaninate, sarcosinate and aspartate.

According to an embodiment of the present invention, the at least one anionic surfactant is a member selected from the group consisting of an isethionate, a taurate, a sarcosinate, a sulfosuccinate, a sulfoacetate, a glycinate, a glutamate and a carboxylate, wherein the at least one anionic surfactant has an alkyl chain from C₈ to C₂₀, and a solubilizing counter cation selected from sodium, potassium and ammonium.

According to an embodiment of the present invention, the at least one anionic surfactant is a taurate, the taurate having an alkyl chain from C₈ to C₂₀, and a solubilizing counter cation selected from sodium, potassium and ammonium.

According to an embodiment of the present invention, the at least one anionic surfactant is a member selected from the group consisting of sodium lauryl methyl isethionate, sodium methyl oleoyl taurate, sodium N-myristoyl-N-methyltaurate, sodium coconut oil fatty acid methyltaurate, and sodium laurylmethyltaurate.

According to an embodiment of the present invention, the anionic surfactant is sodium methyl stearoyl taurate.

According to an embodiment, the anionic surfactant (c) is present in an amount ranging from 0.05% to 3% by weight, and preferably from 0.1% to 1% by weight, relative to the total weight of the composition.

For the purpose of the present invention, the weight ratio of the at least one solid fatty alcohol (b) and at least one anionic surfactant (c) is 4-15 to 40, preferably from 15 to 35, and more preferably from 15 to 35.

The inventors found that, thanks to the specific weight ratio of each component as described above, the composition of the present invention possesses a desired optical/haze/blurring effect. The haze value of the composition is greater than or equal to 30, more preferably greater than or equal to 35.

The haze value is measured by using Haze meter. In particular, the haze value is measured by the machine Haze-gard Plus sold by the company BYK.

The measurement is conducted by preparing a film the composition of the present invention with a thickness of 25µm using the machine Elcometer 4340 Automatic Film Applicator on a transparent film (BYK Byko-charts clear polyester-2mil #2871). The measurement is repeated 5 times to obtain an average haze value.

According to a preferred embodiment, the composition according to the present invention has haze (blurring) effect without commonly used raw material with high haze (blurring) effect, such as whitening charges or silicon lassoers, thus dry squeaky or artificial slippery sensation is avoided.

### α-crystalline phase

The composition of the present invention comprises an α-crystalline phase, or also called as α-gel.

α-crystalline phase, or α-gel, has a hydrate-type crystal structure, which is a lamellar structure. Such structure enables the composition of the present invention a satisfied hydration effect to the keratin materials, in particular the skin.

The present invention makes it possible to have available particularly stable emulsions having small droplets of oily phase coated with a monolamellar layer and/or with an oligolamellar layer which is extremely thin.

Oligolamellar layer is understood to mean a layer comprising from 2 to 5 lipid lamellar (lipid lamellar is understood to mean a membrane of lipid bilayer type as described in the field of liposomes, for example).

### Diameter D[3,2] of the α-crystalline phase according to the present invention

For the purpose of the present invention, the α-crystalline phase of the composition has a diameter D[3,2] ranging from 1µm to 35 µm. Preferably, the diameter of the α-crystalline phase of the composition ranges from 3 µm to 25 µm.

The diameter D [3,2] according to the present invention may be measured by static light scattering using a commercial granulometer such as the MasterSizer 3000 machine from Malvern.

In particular, the diameter D[3,2] is measured by the following protocol:
diluting the composition according to the present invention to 10% in water. The dilution process can be adjusted according to the viscosity of the composition to be measured by a man skilled in the art;
stirring at a speed of 1800 rpm/s;
measuring the D[3,2] of the diluted composition prepared according to the above steps, using MasterSizer 3000;
repeating the above steps for 6 times to obtain an average value D[3,2].
Aqueous phase

The composition of the present invention comprises at least one continuous aqueous phase.

According to an embodiment, the aqueous phase includes water, as the case may be, in a mixture with water-soluble additives and/or solvents.

The aqueous phase may also comprise organic solvents miscible with water (at room temperature - 25°C) such as for example monoalcohols having from 2 to 6 carbon atoms such as ethanol, isopropanol; polyols notably having from 2 to 20 carbon atoms, preferably from 2 to 10 carbon atoms, and preferentially having from 2 to 6 carbon atoms, such as glycerol, propylene glycol, butylene glycol, pentylene glycol, hexylene glycol, caprylylglycol, dipropylene glycol, diethylene glycol; glycol ethers (notably having from 3 to 16 carbon atoms) such as mono-, di- or tri- propylene glycol (C₁-C₄)alkyl ethers, mono-, di- or tri-ethylene glycol (C₁-C₄) alkyl ethers and mixtures thereof.

According to an embodiment, the aqueous phase of the compositions of the present invention comprises a polyol notably glycerol, caprylylglycol or propylene glycol, and a monoalcohol, notably ethanol.

The amount of aqueous phase may range, for example, from 70% to 97% by weight, preferably from 75% to 95% by weight relative to the total weight of the composition. According to an embodiment, the amount of water in the compositions according to the present invention ranges from 70% to 97% by weight, preferably from 75% to 95% by weight, relative to the total weight of the composition.

Preferably, the compositions of the present invention comprise more than 80% by weight of water relative to the total weight of the composition.

The aqueous phase may also contain other additives such as water-soluble active ingredients, preservatives, salts, gelling agents, additional fillers, additional water-soluble or water-dispersible polymers, water-soluble dyes, and so on.

Examples of gelling agents are:
∘ modified or unmodified carboxyvinyl polymers, such as the products sold under the Carbopol (INCI name: carbomer) and Pemulen (INCI name: Acrylates/C10-30 alkyl acrylate crosspolymer) names by Goodrich;
∘ polyacrylamides and polymers and copolymers of 2-acrylamido-2-methylpropanesulphonic acid which are optionally crosslinked and/or neutralized, such as the poly(2-acrylamido-2-methylpropanesulphonic acid) sold by Clariant under the name "Hostacerin AMPS" (INCI name: ammonium polyacryloyldimethyl taurate); or crosslinked anionic copolymers of acrylamide and of AMPS which are provided in the form of an emulsion, such as those sold under the name of Sepigel 305 (CTFA name: Polyacrylamide/C13-14 Isoparaffin/Laureth-7), under the name of Simulgel 600 (CTFA name: Acrylamide/Sodium Acryloyldimethyl Taurate Copolymer/Isohexadecane/Polysorbate 80) by SEPPIC or under the name of Simulgel EG (CTFA name: Sodium Acrylate/Sodium Acryloyldimethyl Taurate Copolymer and Isohexadecane and Polysorbate 80);
∘ acrylate/acrylonitrile copolymers, such as Hypan SS201, sold by Kingston;
∘ polysaccharide biopolymers, such as xanthan gum, guar gum, alginates or modified or unmodified celluloses;
∘ inorganic compounds, such as smectites or modified or unmodified hectorites, such as the Bentone products sold by Rheox, the Laponite products sold by Southern Clay Products or the product Veegum HS sold by R. T. Vanderbilt;
∘ and their mixtures.

Use is preferably made, as hydrophilic gelling agent, of optionally crosslinked polymers and copolymers of 2-acrylamido-2-methylpropanesulphonic acid.

If exists, the gelling agent can be present in the composition in a content (in dry material) ranging for example from 0.001% to 3% by weight, preferably from 0.01% to 1% by weight, relative to the total weight of the composition.

### Oily phase

A composition according to the present invention comprises at least one dispersed oily phase.

According to an embodiment, the oily phase has a refractive index of above 1.43, preferably above 1.50.

It has to be noted that the refractive index mentioned above is a final refractive index of the oily phase. There is no limitation on the refractive index of each oil in the oily phase. The oils chosen to be present in the oily phase of the current invention shall reach the condition that the refractive index of the oil phase is of above 1.43, preferably above 1.50.

According to an embodiment, the composition of the present invention, having an α-crystalline phase, comprising at least one continuous aqueous phase and at least one dispersed oily phase, and comprising:
(a) from 1% to 10% by weight of dicaprylyl carbonate, relative to the total weight of the composition;
(b) from 3% to 10% by weight of at least one linear or branched saturated alcohols having from 14 to 20 carbon atoms, or any mixture thereof, relative to the total weight of the composition; and
(c) from 0.05 to 3% by weight of sodium methyl stearoyl taurate, relative to the total weight of the composition.

### Process for preparing the composition of the present invention

According to one embodiment the composition of the present invention is prepared as follows:
- mixing the aqueous phase containing water and (c) at least one anionic surfactant optionally containing active agents;
- mixing (a) at least one oil and (b) at least one solid fatty alcohol;
- heating separately the mixtures obtained in each of the above steps up to 75-80 °C and maintain the temperature with stirring at 250-500 rev / min until homogeneous;
- mixing the two mixtures under stirring;
- homogenizing the mixture obtained in the previous step at 500-5000 rpm for 10 minutes;
- cooling down to room temperature while stirring.

Lab Homogenization is preferably performed with a high shear mixer, such as those manufactured by Silverson.

Stirring is preferably performed by VMI mixers.

Advantageously, the composition of the present invention is a cosmetic composition for caring for and/or making up keratin materials, and may be in form of lotion, toner, cream, or spray.

The composition of the present invention is stable over time.

"Stable over time" is understood to mean compositions of the present invention which, after storage at all temperatures between 4° C and 45° C for 2 months, do not exhibit any macroscopic change in colour, smell or viscosity, any variation in pH or any variation in microscopic appearance.

### Method and use

The composition of the present invention can be used for a cosmetic process, such as a cosmetic process or method, for making up/caring for the keratin materials, such as the skin, in particular the face and the lips, by being applied to the skin, especially the face.

The present invention also relates to a use of the composition according to the present invention, for making up/caring for the skin, especially for the face.

The present invention relates to a cosmetic process for caring for and/or making up a keratin material, comprising the application, to the keratin material, of at least one composition of the present invention, wherein the keratin material is preferably the skin, in particular the face.

The composition according to the present invention can be used to make the skin anti-finelines during the day. And the composition according to the present invention is easy to spread and penetrate, leaving non-sticky/oily feeling.

The examples that follow are aimed at illustrating the compositions and processes according to the present invention, but are not in any way a limitation of the scope of the present invention.

### EXAMPLES

Example 1 Preparation of Invention compositions and comparative compositon Compositions according to the present invention as well as comparative composition were prepared according to the concentration given in table 1.

**Table 1: Invention compositons 1, 2, and Comparative composition 1**

| **Phase** | **INCI US Name** | **Concentration (% by weight by active ingredient)** | | |
|---|---|---|---|---|
| | | Invention composition | Invention composition | Comparative composition |
| | | **1** | **2** | **1** |
| A | WATER | QS 100 | QS 100 | QS 100 |
| A1 | GLYCERIN (Glycerine 99.7% USP from Acidchem) | 7 | 0 | 7 |
| A2 | SODIUM METHYL STEAROYL TAURATE (NIKKOL SMT from Nikko) | 0.2 | 0,3 | 0.2 |
| B | CETEARYL ALCOHOL (Lanette^{®} O OR from BASF) | 3.0 | 5 | 1.3 |
| B1 | DICAPRYLYL CARBONATE (Cetiol^{®} CC from BASF) | 2.3 | 7.5 | 5 |
| B2 | BUTYROSPERMUM PARKII (SHEA) BUTTER (LIPEX 102 FROM AARHUSKARLSHAMN) | 1.6 | 0 | 0 |
| C | CARBOMER (CARBOPOL 980 POLYMER from LUBRIZOL) | 0.2 | 0 | 0.2 |
| D | SODIUM HYDROXIDE | 0.02 | 0 | 0.02 |
| Weight ratio of ingredient b) and c) | | 15 | 17 | 7 |

Comparative composition 1 comprises less than 3% by weight of solid fatty alcohol.

The Invention compositions and comparative composition were prepared following the steps of:
1. melting phase A and phase B, respectively, in a melter at the temperature of 75-80 °C with stirring at 1000 rpm / min until a homogeneous phase;
2. adding phase B to phase A, using a mixer at 2300 rpm (silverson) for 10 minutes to obtain a homogenous mixture;
3. stirring the mixture using VMI apparatus at a speed of 500 rpm / min, whiling gradually cooling down the mixture to below 60 °C at a speed of 1 °C / minute;
4. adding phase C to phase A+B, while stirring using VMI at 800 rpm / min for 10 minutes, leaving the mixture 1 hour for dispersion;
5. diluting phase D with water, and adding the diluted phase D to the mixture obtained above while stirring using VMI above 1000 rpm / min for 10 minutes;

### Example 2: Evaluation of the Invention compositions and comparative composition

α-crystalline phase (α-gel), the hydrophobic groups of a lamellar liquid crystalline phase are in a molten(liquid) state with a specific sub-lattice structure.

To validate the structure of α-crystalline phase(α-gel), wide-angle X-ray scattering measurement is conducted and α-gel, a sharp peak is observed at 15 nm⁻¹,which corresponds to the diffraction of the sub-lattice.

The D[3.2], haze value, stability and cosmetic property of the compositions according to Invention and the comparative composition were evaluated. The D[3.2] and haze value were evaluated following the method aforementioned.

The stability of the compositions according to invention and the comparative composition were evaluated by leaving the compositions at 45 °C for 2 months, and observing the compositions using microscope under polarized light.

The compositions according to Invention and Comparative composition were given to 10 consumers for application on the facial skin, respectively, and the cosmetic property, such as hydration effect on the skin was scored by the consumers.

In particular, when the skin, after application of the compositions, looks homogeneity, without fine lines, it represents a good hydration effect of the product.

The results of the evaluation are as follow:

| Item | Invention composition | Invention composition | Comparative composition |
|---|---|---|---|
| | **1** | **2** | **1** |
| D[3.2] | 8 | 13 | 30 |
| Haze value | 35 | 64 | 25 |
| Fine Skin | 4.7 | 4.8 | 2.2 |
| Daylong not visible fine lines/ dehydrated lines | 4.5 | 4.7 | 2 |
| Application properties | Easy to spread, fresh feeling, have some blurring effect, slippery, hydrating, moisturizing | Easy to spread, fresh, strong blurring effect, slippery, hydrating, moisturizing | Easy to spread, fresh feeling, not obvious blurring effect, not moisturizing |
| Stability | Stable | Stable | Stable |

Fine skin and daylong not visible fine lines/ dehydrated lines are scored according to the following (score from 5 to 1, higher=better)

| | Strongly agree | Relatively agree | Acceptable | Relatively not agree | Strongly not agree |
|---|---|---|---|---|---|
| Fine skin, or Daylong not visible fine lines/ dehydrated lines | 5 | 4 | 3 | 2 | 1 |

All the above listed compositions are stable over 2 months under the temperature of 45 °C, with no change both in terms of macro- or micro- appearance.

According to the results above, it is obvious that the compositions according to invention both have improved haze (blurring) effects, and such effects are stable over 2 months, under 45 °C. Besides, the compositions according to the present invention present good hydration effect upon application, comparing to the comparative composition.

## Claims

1. A composition having an α-crystalline phase, comprising at least one continuous aqueous phase and at least one dispersed oily phase, and comprising:
(a) at least one oil;
(b) greater than or equal to 3% by weight of at least one solid fatty alcohol, relative to the total weight of the composition; and
(c) at least one anionic surfactant of fatty acid amide sulfonate salts,
wherein the weight ratio of the solid fatty alcohol (b)/the anionic surfactant (c) ranges from 15 to 40,
and the α-crystalline phase having a surface area moment mean diameter D[3,2] ranging from 1µm to 35µm as measured by the method according to the description.

2. The composition of claim 1, wherein the oil (a) is selected from hydrocarbon oils, preferably non-volatile hydrocarbon oils, more preferably selected from non-volatile hydrocarbon oils of animal origin, non-volatile hydrocarbon oils of plant origin, linear or branched non-volatile hydrocarbon oils of mineral or synthetic origin, synthetic ethers having from 10 to 40 carbon atoms, oils of formula R₁COOR₂, in which R₁ represents a linear or branched fatty acid residue containing from 1 to 40 carbon atoms, and R₂ represents a hydrocarbon-based chain, polyol esters and pentaerythritol esters, esters of diol dimers and diacid dimers, copolymers of a diol dimer and of a diacid dimer and esters thereof, copolymers of polyols and of diacid dimers, and esters thereof, fatty alcohols that are liquid at ambient temperature, C₁₂-C₂₂ fatty acids, dialkyl carbonates, oils of higher molar mass having in particular a molar mass ranging from approximately 400 to approximately 10,000 g/mol, or a mixture thereof.

3. The composition of claim 1 or 2, wherein the oil (a) is a dialkyl carbonate of following formula (1): 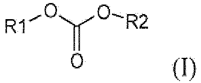
wherein R₁ and R₂, identical or different, each represents a linear or branched hydrocarbon chain comprising from 3 to 30 carbon atoms;
preferably in formula (1), R₁ and R₂, which are identical, represent a linear hydrocarbon chain comprising from 3 to 8 carbon atoms;
more preferably, the dialkyl carbonate is dicaprylyl carbonate.

4. The composition of any one of the preceding claims 1 to 3, wherein the oil is present in an amount ranging from 0.1% to 15% by weight, preferably ranging from 1% to 10% by weight, relative to the total weight of the composition.

5. The composition of any one of the preceding claims 1 to 4, wherein the solid fatty alcohol (b) is selected from the fatty alcohol having from 14 to 30 carbon atoms, preferably from 14 to 22 carbon atoms; more preferably selected from the group consisting of myristyl alcohol, cetyl alcohol, palmitoleyl alcohol, stearyl alcohol , arachidyl alcohol, behenyl alcohol, erucyl alcohol, or a mixture thereof; more preferably selected from cetyl alcohol, stearyl alcohol, arachidyl alcohol, behenyl alcohol, or a mixture thereof.

6. The composition of any one of the preceding claims 1 to 5. wherein the solid fatty alcohol is present in an amount greater than or equal to 3% by weight, preferably from 3% to 15% by weight, more preferably from 3% to 10% by weight, relative to the total weight of the composition.

7. The composition of any one of the preceding claims 1 to 6, wherein the surfactant (c) is a amino acid surfactant of formula (II): 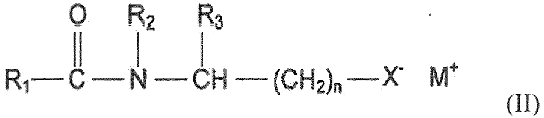 wherein:
R₁ is a saturated or unsaturated, straight or branched alkyl chain with 7 to 17 carbon atoms atoms,
R₂ is H or a methyl,
R₃ is H, COO- M⁺, CH₂COO- M or COOH,
n is 0 to 2,
X is SO₃ ⁻ and
M is independent from each other H, sodium or potassium and a sorbitan surfactant;
more preferably, the anionic surfactant is a taurate surfactant of formula (III) 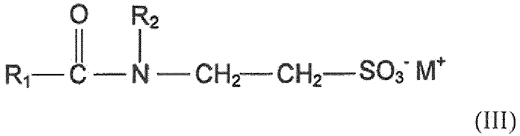
wherein
R₁ is preferably a saturated or unsaturated, straight or branched alkyl chain with 7 to 17 carbon atoms, and more preferably 9 to 13 carbon atoms,
R₂ is H or methyl,
and M is H, sodium or potassium.

8. The composition of claim 7, wherein the anionic surfactant is selected from the group consisting of potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, and sodium methyl stearoyl taurate and mixtures thereof, preferred are potassium cocoyl taurate, potassium methyl cocoyl taurate, sodium caproyl methyl taurate, sodium cocoyl taurate, sodium lauroyl taurate, sodium methyl cocoyl taurate and sodium methyl lauroyl taurate and mixtures thereof, more preferred are potassium cocoyl taurate, potassium methyl cocoyl taurate, sodium cocoyl taurate, sodium lauroyl taurate, sodium methyl cocoyl taurate and sodium methyl lauroyl taurate and mixtures thereof, preferably sodium methyl stearoyl taurate.

9. The composition of any one of the preceding claims 1 to 8, wherein the anionic surfactant (c) is present in an amount ranging from 0.05% to 3% by weight, and preferably from 0.1% to 1% by weight, relative to the total weight of the composition.

10. The composition of any one of the preceding claims 1 to 9, wherein the weight ratio of the solid fatty alcohol (b) and the anionic surfactant (c) is from 15 to 35.

11. The composition of any one of the preceding claims 1 to 10, wherein the α-crystalline phase has a surface area moment mean diameter D[3,2] ranging from 1 µm to 35 µm, preferably ranges from 3 µm to 25 µm.

12. The composition of any one of the preceding claims 1 to 11, wherein the oily phase has a refractive index of above 1.43, preferably above 1.50.

13. The composition of claim 1, comprising:
(a) from 1% to 10% by weight of dicaprylyl carbonate, relative to the total weight of the composition;
(b) from 3% to 10% by weight of at least one linear or branched saturated alcohols having from 14 to 20 carbon atoms, or any mixture thereof, relative to the total weight of the composition; and
(c) from 0.05 to 3% by weight of sodium methyl stearoyl taurate, relative to the total weight of the composition.

14. A cosmetic process for caring for and/or making up a keratin material, comprising the application, to the keratin material, of at least one composition of any one of the preceding claims 1 to 13, wherein the keratin material is preferably the skin, in particular the face.

## Patentansprüche

1. Zusammensetzung mit einer α-kristallinen Phase, umfassend mindestens eine kontinuierliche wässrige Phase und mindestens eine dispergierte Ölphase, und umfassend:
(a) mindestens ein Öl;
(b) größer oder gleich 3 Gew.-% mindestens eines festen Fettalkohols, bezogen auf das Gesamtgewicht der Zusammensetzung; und
(c) mindestens ein anionisches Tensid von Fettsäureamidsulfonatsalzen, wobei das Gewichtsverhältnis des festen Fettalkohols (b) zu dem anionischen Tensid (c) im Bereich von 15 bis 40 liegt,
und wobei die α-kristalline Phase einen mittleren Durchmesser D [3,2] des Oberflächenmoments im Bereich von 1 µm bis 35 µm hat, gemessen mit dem Verfahren gemäß der Beschreibung.

2. Zusammensetzung nach Anspruch 1, wobei das Öl (a) ausgewählt ist aus Kohlenwasserstoffölen, vorzugsweise nichtflüchtigen Kohlenwasserstoffölen, mehr bevorzugt ausgewählt ist aus nichtflüchtigen Kohlenwasserstoffölen tierischen Ursprungs, nichtflüchtigen Kohlenwasserstoffölen pflanzlichen Ursprungs, linearen oder verzweigten nichtflüchtigen Kohlenwasserstoffölen mineralischen oder synthetischen Ursprungs, synthetischen Ethern mit 10 bis 40 Kohlenstoffatomen, Ölen der Formel R₁COOR₂, in der R₁ einen linearen oder verzweigten Fettsäurerest repräsentiert, der 1 bis 40 Kohlenstoffatome enthält, und R₂ eine kohlenwasserstoffbasierte Kette, Polyolester und Pentaerythritolester, Ester von Dioldimeren und Disäuredimeren, Copolymere eines Dioldimers und eines Disäuredimers und Ester davon, Copolymere von Polyolen und von Disäuredimeren und Ester davon, Fettalkohole, die bei Umgebungstemperatur flüssig sind, C₁₂-C₂₂-Fettsäuren, Dialkylcarbonate, Öle von höherer Molmasse mit insbesondere einer Molmasse im Bereich von ungefähr 400 bis ungefähr 10.000 g/mol oder eine Mischung davon repräsentiert.

3. Zusammensetzung nach Anspruch 1 oder 2, wobei das Öl (a) ein Dialkylcarbonat der folgenden Formel (I) ist: 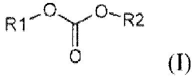
worin R₁ und R₂ identisch oder verschieden sind und jeweils eine lineare oder verzweigte Kohlenwasserstoffkette mit 3 bis 30 Kohlenstoffatomen repräsentieren; vorzugsweise repräsentieren R₁ und R₂, die identisch sind, in Formel (I) eine lineare Kohlenwasserstoffkette mit 3 bis 8 Kohlenstoffatomen;
mehr bevorzugt ist das Dialkylcarbonat Dicaprylylcarbonat.

4. Zusammensetzung nach einem der vorhergehenden Ansprüche 1 bis 3, wobei das Öl in einer Menge im Bereich von 0,1 Gew.-% bis 15 Gew.-%, vorzugsweise im Bereich von 1 Gew.-% bis 10 Gew.-% vorliegt, bezogen auf das Gesamtgewicht der Zusammensetzung.

5. Zusammensetzung nach einem der vorhergehenden Ansprüche 1 bis 4, wobei der feste Fettalkohol (b) ausgewählt ist aus dem Fettalkohol mit 14 bis 30 Kohlenstoffatomen, vorzugsweise mit 14 bis 22 Kohlenstoffatomen; mehr bevorzugt ausgewählt aus der aus Myristylalkohol, Cetylalkohol, Palmitoleylalkohol, Stearylalkohol, Arachidylalkohol, Behenylalkohol, Erucylalkohol oder einer Mischung davon bestehenden Gruppe; mehr bevorzugt ausgewählt aus Cetylalkohol, Stearylalkohol, Arachidylalkohol, Behenylalkohol oder einer Mischung davon.

6. Zusammensetzung nach einem der vorhergehenden Ansprüche 1 bis 5, wobei der feste Fettalkohol in einer Menge größer oder gleich 3 Gew.-% vorliegt, vorzugsweise von 3 Gew.-% bis 5 Gew.-%, mehr bevorzugt von 3 Gew.-% bis 10 Gew.-%, bezogen auf das Gesamtgewicht der Zusammensetzung.

7. Zusammensetzung nach einem der vorhergehenden Ansprüche 1 bis 6, wobei das Tensid (c) ein Aminosäuretensid der Formel (II) ist: 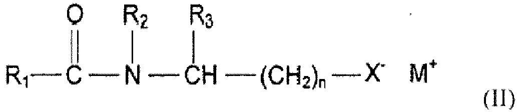 worin:
R₁ eine gesättigte oder ungesättigte, gerade oder verzweigte Alkylkette mit 7 bis 17 Kohlenstoffatomen ist,
R₂ H oder ein Methyl ist,
R₃ H, COO- M⁺, CH₂COO- M oder COOH ist,
n 0 bis 2 ist,
X SO₃⁻ ist, und
M unabhängig voneinander H, Natrium oder Kalium und ein Sorbitantensid ist;
mehr bevorzugt ist das anionische Tensid ein Taurattensid der Formel (III): 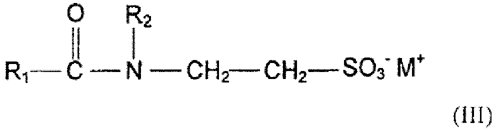 worin
R₁ vorzugsweise eine gesättigte oder ungesättigte, gerade oder verzweigte Alkylkette mit 7 bis 17 Kohlenstoffatomen und mehr bevorzugt mit 9 bis 13 Kohlenstoffatomen ist,
R₂ H oder Methyl ist,
und M H, Natrium oder Kalium ist.

8. Zusammensetzung nach Anspruch 7, wobei das anionische Tensid ausgewählt ist aus der aus Kaliumcocoyltaurat, Kaliummethylcocoyltaurat, Natriumcaproylmethyltaurat, Natriumcocoyltaurat, Natriumlauroyltaurat, Natriummethylcocoyltaurat, Natriummethyllauroyltaurat, Natriumethylmyristoyltaurat, Natriummethyloleoyltaurat, Natriummethylpalmitoyltaurat und Natriummethylstearoyltaurat und Mischungen davon bestehenden Gruppe; bevorzugt sind Kaliumcocoyltaurat, Kaliummethylcocoyltaurat, Natriumcaproylmethyltaurat, Natriumcocoyltaurat, Natriumlauroyltaurat, Natriummethylcocoyltaurat und Natriummethyllauroyltaurat und Mischungen davon; mehr bevorzugt sind Kaliumcocoyltaurat, Kaliummethylcocoyltaurat, Natriumcocoyltaurat, Natriumlauroyltaurat, Natriummethylcocoyltaurat und Natriummethyllauroyltaurat und Mischungen davon; vorzugsweise Natriummethylstearoyltaurat.

9. Zusammensetzung nach einem der vorhergehenden Ansprüche 1 bis 8, wobei das anionische Tensid (c) in einer Menge im Bereich von 0,05 Gew.-% bis 3 Gew.-% und vorzugsweise von 0,1 Gew.-% bis 1 Gew.-% vorliegt, bezogen auf das Gesamtgewicht der Zusammensetzung.

10. Zusammensetzung nach einem der vorhergehenden Ansprüche 1 bis 9, wobei das Gewichtsverhältnis des festen Fettalkohols (b) und des anionischen Tensids (c) von 15 bis 35 beträgt.

11. Zusammensetzung nach einem der vorhergehenden Ansprüche 1 bis 10, wobei die α-kristalline Phase einen mittleren Durchmesser D [3,2] des Oberflächenmoments im Bereich von 1 µm bis 35 µm, vorzugsweise im Bereich von 3 µm bis 25 µm hat.

12. Zusammensetzung nach einem der vorhergehenden Ansprüche 1 bis 11, wobei die Ölphase einen Brechungsindex von über 1,43, vorzugsweise über 1,50 hat.

13. Zusammensetzung nach Anspruch 1, umfassend:
(a) von 1 Gew.-% bis 10 Gew.-% Dicaprylylcarbonat, bezogen auf das Gesamtgewicht der Zusammensetzung;
(b) von 3 Gew.-% bis 10 Gew.-% mindestens eines linearen oder verzweigten gesättigten Alkohols mit 14 bis 20 Kohlenstoffatomen, oder eine Mischung davon, bezogen auf das Gesamtgewicht der Zusammensetzung; und
(c) von 0,05 Gew.-% bis 3 Gew.-% Natriummethylstearoyltaurat, bezogen auf das Gesamtgewicht der Zusammensetzung.

14. Kosmetisches Verfahren zur Pflege und/oder zum Schminken eines Keratinmaterials, bei dem auf das Keratinmaterial mindestens eine Zusammensetzung nach einem der vorhergehenden Ansprüche 1 bis 13 aufgebracht wird, wobei das Keratinmaterial vorzugsweise die Haut, insbesondere das Gesicht ist.

## Revendications

1. Composition ayant une phase cristalline α, comprenant au moins une phase aqueuse continue et au moins une phase huileuse dispersée, et comprenant :
(a) au moins une huile ;
(b) supérieur ou égal à 3 % en poids d'au moins un alcool gras solide, par rapport au poids total de la composition ; et
(c) au moins un tensioactif anionique de sels d'amide-sulfonate d'acide gras,
dans laquelle le rapport en poids alcool gras solide (b)/tensioactif anionique (c) va de 15 à 40,
et la phase cristalline α présentant un diamètre moyen de moment de surface D[3,2] allant de 1 um à 35 µm tel que mesuré par le procédé selon la description.

2. Composition selon la revendication 1, dans laquelle l'huile (a) est sélectionnée parmi les huiles d'hydrocarbures, de préférence les huiles d'hydrocarbures non volatiles, de manière davantage préférée sélectionnées parmi les huiles d'hydrocarbures non volatiles d'origine animale, les huiles d'hydrocarbures non volatiles d'origine végétale, les huiles d'hydrocarbures non volatiles linéaires ou ramifiées d'origine minérale ou synthétique, les éthers de synthèse ayant de 10 à 40 atomes de carbone, les huiles de formule R₁COOR₂, dans laquelle R₁ représente un résidu d'acide gras linéaire ou ramifié contenant de 1 à 40 atomes de carbone, et R₂ représente une chaîne hydrocarbonée, les esters de polyol et les esters de pentaérythritol, les esters de dimères de diol et de dimères de diacide, les copolymères d'un dimère de diol et d'un dimère de diacide et les esters de ceux-ci, les copolymères de polyols et de dimères de diacide, et les esters de ceux-ci, les alcools gras qui sont liquides à température ambiante, les acides gras en C₁₂-C₂₂, les carbonates de dialkyle, les huiles de masse molaire supérieure présentant en particulier une masse molaire allant d'approximativement 400 à approximativement 10 000 g/mol, ou un mélange de ceux-ci.

3. Composition selon la revendication 1 ou 2, dans laquelle l'huile (a) est un carbonate de dialkyle de formule (I) suivante : 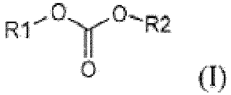
dans laquelle R₁ et R₂, identiques ou différents, représentent chacun une chaîne hydrocarbonée linéaire ou ramifiée comprenant de 3 à 30 atomes de carbone ;
de préférence dans la formule (I), R₁ et R₂, qui sont identiques, représentent une chaîne hydrocarbonée linéaire comprenant 3 à 8 atomes de carbone ;
de manière davantage préférée, le carbonate de dialkyle est le carbonate de dicaprylyle.

4. Composition selon l'une quelconque des revendications 1 à 3 précédentes, dans laquelle l'huile est présente en une quantité allant de 0,1 % à 15 % en poids, de préférence allant de 1 % à 10 % en poids, par rapport au poids total de la composition.

5. Composition selon l'une quelconque des revendications 1 à 4 précédentes, dans laquelle l'alcool gras solide (b) est sélectionné parmi les alcools gras ayant de 14 à 30 atomes de carbone, de préférence de 14 à 22 atomes de carbone ; de manière davantage préférée sélectionné dans le groupe consistant en l'alcool myristylique, l'alcool cétylique, l'alcool palmitoléylique, l'alcool stéarylique, l'alcool arachidylique, l'alcool béhénylique, l'alcool érucylique, ou un mélange de ceux-ci ; de manière davantage préférée sélectionné parmi l'alcool cétylique, l'alcool stéarylique, l'alcool arachidylique, l'alcool béhénylique, ou un mélange de ceux-ci.

6. Composition selon l'une des revendications 1 à 5 précédentes, dans laquelle l'alcool gras solide est présent en une quantité supérieure ou égale à 3 % en poids, de préférence de 3 % à 15 % en poids, de manière davantage préférée de 3 % à 10 % en poids, par rapport au poids total de la composition.

7. Composition selon l'une des revendications 1 à 6 précédentes, dans laquelle le surfactant (c) est un surfactant à acide aminé de formule (II) : 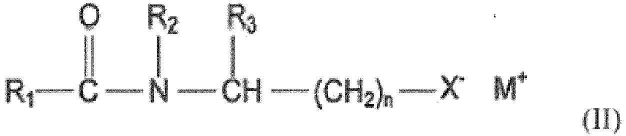 dans laquelle :
R₁ est une chaîne alkyle saturée ou insaturée, linéaire ou
ramifiée ayant 7 à 17 atomes de carbone,
R₂ est H ou un méthyle,
R₃ est H, COO- M⁺, CH₂COO- M ou COOH,
n est 0 à 2,
X est SO₃⁻ et
M est, indépendamment les uns des autres, H, un sodium ou un potassium et un surfactant à sorbitane ;
de manière davantage préférée, le tensioactif anionique est un tensioactif à taurate de formule (III) 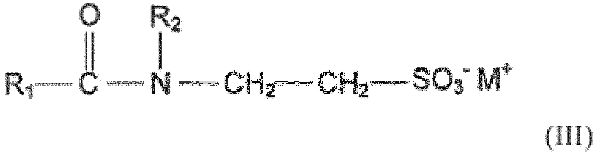
dans laquelle
R₁ est de préférence une chaîne alkyle saturée ou insaturée, linéaire ou ramifiée ayant 7 à 17 atomes de carbone, et de manière davantage préférée 9 à 13 atomes de carbone,
R₂ est H ou un méthyle,
et M est H, un sodium ou un potassium.

8. Composition selon la revendication 7, dans laquelle le tensioactif anionique est sélectionné dans le groupe consistant en le cocoyltaurate de potassium, le méthylcocoyltaurate de potassium, le caproylméthyltaurate de sodium, le cocoyltaurate de sodium, le lauroyltaurate de sodium, le méthylcocoyltaurate de sodium, le méthyllauroyltaurate de sodium, le méthylmyristoyltaurate de sodium, le méthyloléoyltaurate de sodium, le méthylpalmitoyltaurate de sodium, et le méthylstéaroyltaurate de sodium et les mélanges de ceux-ci, sont préférés le cocoyltaurate de potassium, le méthylcocoyltaurate de potassium, le caproylméthyltaurate de sodium, le cocoyltaurate de sodium, le lauroyltaurate de sodium, le méthylcocoyltaurate de sodium et le méthyllauroyltaurate de sodium et les mélanges de ceux-ci, sont davantage préférés le cocoyltaurate de potassium, le méthylcocoyltaurate de potassium, le cocoyltaurate de sodium, le lauroyltaurate de sodium, le méthylcocoyltaurate de sodium et le méthyllauroyltaurate de sodium et les mélanges de ceux-ci ; de préférence le méthylstéaroyltaurate de sodium.

9. Composition selon l'une quelconque des revendications 1 à 8 précédentes, dans laquelle le tensioactif anionique (c) est présent en une quantité allant de 0,05 % à 3 % en poids, et de préférence de 0,1 % à 1 % en poids, par rapport au poids total de la composition.

10. Composition selon l'une quelconque des revendications 1 à 9 précédentes, dans laquelle le rapport en poids de l'alcool gras solide (b) et du tensioactif anionique (c) est de 15 à 35.

11. Composition selon l'une quelconque des revendications 1 à 10 précédentes, dans laquelle la phase cristalline α présente un diamètre moyen de moment de surface D[3,2] allant de 1 um à 35 um, de préférence de 3 um à 25 µm.

12. Composition selon l'une quelconque des revendications 1 à 11 précédentes, dans laquelle la phase huileuse présente un indice de réfraction supérieur à 1,43, de préférence supérieur à 1,50.

13. Composition selon la revendication 1, comprenant :
(a) de 1 % à 10 % en poids de carbonate de dicaprylyle, par rapport au poids total de la composition ;
(b) de 3 % à 10 % en poids d'au moins un alcool saturé linéaire ou ramifié ayant de 14 à 20 atomes de carbone, ou n'importe quel mélange de ceux-ci, par rapport au poids total de la composition ; et
(c) de 0,05 à 3 % en poids de méthylstéaroyltaurate de sodium, par rapport au poids total de la composition.

14. Procédé cosmétique de soin et/ou de maquillage d'une matière kératinique, comprenant l'application, sur la matière kératinique, d'au moins une composition selon l'une quelconque des revendications 1 à 13 précédentes, dans lequel la matière kératinique est de préférence la peau, en particulier le visage.
